# EUROPEAN PATENT SPECIFICATION

(11) **EP 1 550 459 B1**
(45) Date of publication and mention of the grant of the patent: **02.01.2013**
(21) Application number: 03730607.3
(22) Date of filing: 23.05.2003
(51) Int. Cl.: A61K 8/44, A61Q 19/00

(54) **PARAKERATOSIS INHIBITOR, PORE-SHRINKING AGENT AND SKIN PREPARATION FOR EXTERNAL USE**
PARAKERATOSE-HEMMER, PORENSCHRUMPFENDES MITTEL UND HAUTPRÄPARAT FÜR DIE TOPISCHE ANWENDUNG
INHIBITEUR DE LA PARAKERATOSE, AGENT DE RESSERREMENT DES PORES ET PREPARATION DE LA PEAU AUX FINS D'UTILISATION EXTERNE

(30) Priority: 28.05.2002 JP 2002153457
(43) Date of publication of application: 06.07.2005
(73) Proprietor: SHISEIDO COMPANY LIMITED, Chuo-ku, Tokyo 104-8010 (JP)
(72) Inventor: KATSUTA, Yuji, Shiseido Research Center, Yokohama-shi, Kanagawa 224-8558 (JP); INOMATA, Shinji, Shiseido Research Center, Yokohama-shi, Kanagawa 224-8558 (JP)
(74) Representative: Merkle, Gebhard
(86) International application number: PCT/JP2003/006467
(87) International publication number: WO 2003/099327

(56) References cited:
- EP-A1- 0 413 528
- EP-A1- 0 722 736
- EP-A1- 0 749 752
- WO-A-97/10815
- WO-A-99/13819
- WO-A1-01/24823
- WO-A1-97/09987
- WO-A1-97/35618
- WO-A1-98/11886
- WO-A1-03/053466
- WO-A2-02/05834
- WO-A2-02/11677
- WO-A2-98/08546
- JP-A- 11 060 469
- JP-A- 11 158 055
- JP-A- 2002 316 929
- US-A- 5 874 420
- GENEVER P G ET AL: "Evidence for a novel glutamate-mediated signaling pathway in keratinocytes" JOURNAL OF INVESTIGATIVE DERMATOLOGY, NEW YORK, NY, US, vol. 112, no. 3, 1999, pages 337-342, XP002966361 ISSN: 0022-202X
- TOSHIYUKI IIDA ET AL.: 'Hito hobu nokeana no medachi to kakuka ijo' JAPANESE DERMATOLOGICAL ASSOCIATION ZASSHI vol. 113, no. 5, 30 April 2003, page 846, XP002972171
- LOWITT M.H. ET AL.: 'Cuteneous eruptions from suramin' ARCH. DERMATOL. vol. 131, no. 10, 1995, pages 1147 - 1153, XP002972172

## Description

### Technical Field

The invention relates to a parakeratosis inhibitor that inhibits parakeratosis caused by sebum. In particular, the invention relates to a pore-shrinking agent that maintains normal skin conditions around the pore and suppresses a conical structure of the pore from becoming conspicuous by inhibiting parakeratosis caused by stimulatory components in the sebum around the pore. More particularly, the invention relates to a parakeratosis inhibitory skin preparation for external use and a pore-shrinking skin preparation for external use.

### Background Art

Hitherto, many people have worried about conspicuous pores and have demanded a skin preparation for external use for making the pore inconspicuous. However, the mechanism for making the pore conspicuous has not been elucidated yet, and use of an astringent cosmetics and excision of parakeratosis have been usual treatments of parakeratosis. However, the object of use of the astringent cosmetics is to tighten the skin, and the action thereof is to temporarily reduce the temperature of the skin surface with an alcohol, or to coagulate proteins with organic acids and the like. Accordingly, the skin suffers a great burden since the skin is temporarily tightened without fundamentally solving the problem of conspicuous pores, and the effect of the astringent cosmetics has been insufficient.

Excision of keratin plug is to physically remove the keratin plug by which the skin is often damaged by a physical force, and side effects on the skin have been a serious problem. The effect of this method is not always satisfactory since the effect thereof is temporary and keratin plug is readily regenerated, and removing keratin plug may only expand the pore.

Accordingly, developments of a skin preparation for external use that is safe and burdens a small load on the skin, and has a large effect for improving conspicuous pores have been desired.

The object of the invention performed based on the circumstances above is to provide a substance having a pore-shrinking function, and a preparation such as a skin preparation for external use for improving conspicuous pores by elucidating the mechanism for making the pores conspicuous.

WO 97/10815 A discloses antagonists of the NMDA receptor, anticholinergic agents and sympathetic blocking agents used for treating pain.

WO 99/13819 A discloses a topical cosmetic or pharmaceutical composition comprising an effective amount of an anti-stinging amino acid selected from the group consisting of an amino butyric acid, glutamine or glycine.

US 5,874,420 discloses P2X purinoceptor receptor (an ATP receptor) antagonists and their use for regulating vagal tone.

### Disclosure of the Invention

The inventors of the invention have made intensive studies for solving the problems above starting from the study of the mechanism of generating the conspicuous pores.

Epidermal keratinocyte proliferates in the basal layer, and moves to the surface layer to mature there into a keratin layer. Nuclei in the cell disappear when epidermal cells turns into the keratin layer, and the cells are flattened. However, some of the epidermal keratinocyte remain in the keratin layer as immature cells having the nucleus in the cell, which is called as parakeratosis. Parakeratosis causes stratified ablation of the keratin layer, which results in expansion of the pore. The conspicuous pores are formed by the conical structure of infundibulum portions as well as of the portions around the pore (the portion of the infundibulum having keratin plug). The skin state is poor in the conical structure portion around the pore to readily cause parakeratosis that expands the pore.

Accordingly, since the mechanism of forming the conspicuous pores is based on parakeratosis caused by the sebum, a parakeratosis-inhibitory substance is effective for shrinking the pore. It was elucidated that the conical structure around the pore is diminished by improving parakeratosis, or the pore shrinks and conspicuous pores are improved by inhibiting parakeratosis.

The inventors of the invention found that oleic acid that is an excitatory component in the sebum and induces parakeratosis has an action for exciting cells such as epidermal keratinocyte (or for increasing the concentration of calcium), and that an agonist to an excitatory cell receptor and an antagonist to an inhibitory cell receptor have an action for worsening parakeratosis.

The inventors of the invention found that the problems above are solved by providing a novel parakeratosis inhibitor and pore-shrinking agent having a parakeratosis inhibitory function and pore-shrinking function based on the discoveries above, and have proceeded the investigation.

The inventors of the invention attempted surveillance of compounds having a parakeratosis inhibitory function and pore-shrinking function, and found that an antagonist to the excitatory cell receptor and an agonist to the inhibitory cell receptor have desirable functions as described above. The invention have been completed based on these discoveries.

It is out of the sphere of information available for the inventors of the invention that the antagonist and agonist have the parakeratosis inhibitory function, or such substances exhibit a conical structure-shrinking action around the pore. The invention provides a parakeratosis inhibitory skin preparation for external use containing an antagonist to the excitatory cell receptor.

The invention also provides a pore-shrinking agent comprising the antagonist to the excitatory cell receptor. The invention further provides a pore-shrinking skin preparation for external use containing the antagonist to the excitatory cell receptor.

Preferable receptors of the antagonist to the excitatory cell receptor used for the parakeratosis inhibitory skin preparation for external use, pore-shrinking agent and pore-shrinking skin preparation for external use are as follows. Glutamic acid receptors such as N-methyl-D-aspartic acid receptor are preferable as the excitatory cell receptors.

The antagonist to the N-methyl-D-aspartic acid receptor is preferably D-glutamic acid.

### Best Mode for Carrying Out the Invention

The embodiments of the invention will be described in detail hereinafter.

An antagonist to the excitatory cell receptor is used for the parakeratosis inhibitor, pore-shrinking agent, parakeratosis inhibitory preparation for external use and pore-shrinking preparation for external use.

The "excitatory cell receptor" as used in the invention refers to an excitatory receptor that leads skin cells, or cells constituting the cornified layer, epidermis, basement membrane and derma, for example the cells existing in the cell membrane of the epidermal keratinocyte, to an excitation state. Such excitation is induced by an influx of Ca²⁺ and Na⁺ ions caused by binding an agonist to the receptors.

Since the glutamic acid receptor, ATP receptor, acetylcholine-nicotinic acid receptor and serotonin receptor have been found in the skin cells today as the excitatory cell receptors, these receptors may be the object of the excitatory cell receptors.

The glutamic acid receptor is preferable as the excitatory cell receptor . N-methyl-D-aspartic acid receptor (abbreviated as NMDA receptor hereinafter) is preferable as the glutamic acid receptor.

Specific antagonists to respective excitatory cell receptors are used. For example, such as dizocylpin (abbreviated as MK-801 hereinafter), D-glutamic acid, D-AP7, conantokin T and (R)-CPP are used as the antagonist to NMDA receptor. Examples of the antagonist to ATP receptor include suramin, pyridoxal phosphate-6-azophenyl-2',4'-disulfonic acid (abbreviated as PPADS hereinafter) and trinitrophenyl-ATP (abbreviated as TNP-ATP hereinafter). Benzoquinonium, condelphine and α-conotoxin-E1 and the like are examples of the antagonist to the acetylcholine-nicotinic acid receptor. MDL-72222, Y-25130 and metoclopramide and the like are the examples of the antagonist to the serotonin receptor.

The preferable antagonists to the excitatory cell receptor of the invention is D-glutamic acid as the antagonist to the NMDA receptor.

The "inhibitory cell receptor" refers to the inhibitory cell receptors that lead the cells that constitute the skin cells, or cells constituting the cornified layer, epiderm, basement membrane and dermis, for example, cells existing in the cell membrane of keratinocyte, from an excitatory state to an inhibitory state. Such inhibition is induced by influx of Cl- ions into the cell as a result of binding of the antagonist to the receptor.

Since γ-aminobutyric acid receptor (abbreviated as GABA receptor hereinafter) and glycine receptor have been found in the skin cells today as the inhibitory cell receptors as described above, they may be the object of the inhibitory cell receptors.

GABA receptor and glycine receptor are among the inhibitory receptors. Cl⁻ channel-involving bicuculline sensitive receptor (abbreviated as GABA receptor type A hereinafter) is used as the GABA receptor.

Specific agonists to respective inhibitory cell receptors are used. For example, the agonists to the GABA receptor type A include such as γ-aminobutyric acid (abbreviated as GABA hereinafter), muscimol, isogubacin, TACA and THIP. Examples of the agonists to the glycine receptor include glycine, β-alanine, hypotaurine, serine and taurine.

The preferable agonists to the inhibitory cell receptor of the invention are GAVA, muscimol, isogubacin as the agonists of GABA receptor of type A, and glycine as the agonist to the glycine receptor.

The antagonists to the excitatory cell receptors and the according to the invention have excellent functions for inhibiting parakeratosis and shrinking of pores as will be proved hereinafter. Accordingly, the antagonists are useful as the parakeratosis inhibitor and pore-shrinking agent.

The composition containing the antagonist to the excitatory cell receptor of the invention, is able to exhibit a parakeratosis inhibitory function and pore-shrinking function of the antagonist to the excitatory cell receptor. Accordingly, such composition can be applied to a skin parakeratosis inhibitor for external use or a skin pore-shrinking agent for external use (named as a composition for external use).

The composition for external use of the invention may be utilized as medicines, quasi-drugs and cosmetics applied to the outer coat such as a pore-shrinking agent, face cosmetics for improving conspicuous pores on the nose and cheek, and body skin treatment agent for external use for improving conspicuous pores after depilation of the leg, particularly as cosmetics. The composition for external use of the invention serves for maintaining healthy state of the skin.

When the antagonist to the excitatory cell preceptor is blended with the composition for external use of the invention, one or at least two of the compounds are arbitrarily selected for use. The content of the antagonist to the excitatory cell receptor of the invention is preferably 0.001 to 20% by mass, more preferably 0.01 to 10.0% by mass, and particularly 0.1 to 5% by mass in the total amount of the conposition for external use. The effect of the invention cannot be sufficiently displayed when the content is less than 0.001% by mass, while formulation of the preparation is difficult when the content exceeds 20.0% by mass. Not so large effect can be expected by blending the preparation in a proportion exceeding 10.0% by mass.

The composition for external use of the invention may be manufactured according to the conventional method. While only the antagonist to the excitatory cell receptor of the invention may be formulated, components usually used for the skin preparation for external use such as cosmetics and medicines, for example oils, surfactants, powders, colorants, water, humectants, viscosifiers, alcohols, various skin nutrients, antioxidants, UV absorbing agents, perfumes and antiseptics may be appropriately blended.

Other substances that may be appropriately blended include metal blocking agent such as EDTA-2Na, EDTA-3Na, sodium citrate, sodiumpolyphosphate, sodiummetaphosphate and glucuronic acid; caffeine, tannin, verapamil and their derivatives; Licorice extract, glabridin, hot water extract of Kakyoku, various Chinese herb medicine, tocopherol acetate, glycyrrhizic acid, derivatives or salts thereof; whitening agents such as vitamin C, magnesium ascorbic acid phosphate, ascorbic acid glucoside, arbutin and Koj ic acid; sugars such as glucose, fructose, mannose, sucrose and trehalose; vitamin A such as lethinol, lethinoic acid, lethinol acetate and lethinol palmitate.

The formulation of the composition of the invention may be in a wide range of forms such as aqueous solution, solubilized, emulsion, powder, oil, gel, ointment, aerosol, water-oil two phase and water-oil-water three phase forms. The formulation may be applicable in various formulations described above such as face cleaning agent, cosmetics, lotions, creams, gel, essence (beauty liquid), pack and mask as fundamental cosmetics. The formulation may be also applied to make-up cosmetics such as foundations, and toiletry products such as body soaps and soaps. The formulation may be also used as quasi-drugs such as various ointments. However, the formulation of the composition for external use of the invention is not restricted to these formulations and forms.

### [Examples]

While the invention is described in detail with reference to examples, the blend ratio is expressed by % by mass, unless otherwise stated.

### [Excitatory action test of cells with oleic acid]

Epidermal keratinocyte was cultivated on an appropriate medium, for example, KGM medium, according to the conventional method. The cultured cells were seeded on a cover glass chamber and cultivated there a day before measuring calcium ions. An appropriate buffer solution, for example BSS (balanced salt solution) and a calcium sensitive fluorescent pigment (fura-2-AM) were added in the cultured cell at a concentration of about 2 µM on the next day, and the fluorescent pigment was allowed to be incorporated into the cell by incubating under an appropriate condition (for example 30 minutes at 37°C). After completing intake of the pigment, the same buffer solution (fresh BSS) was added. The same buffer solution (BSS) dissolving a test substance (oleic acid) was added to the culture thereafter to measure the calcium ion concentration in the cell. The same measurement was carried out by adding only the same buffer solution (BSS). The results are shown in Table 1. The fluorescence intensity at 340 nm was divided by the fluorescence intensity at 380 nm for determining the calcium concentration according to the conventional method.

**Table 1**

| Sample Concentration | Change of Calcium Ion Concentration in the Cell (Change of Fluorescence Intensity Ratio) Average ± SE |
|---|---|
| Reference Buffer Solution | 0.051 ± 0.007 |
| Oleic Acid (50 µM) | 0.304 ± 0.038 |

The results in Table 1 show that oleic acid as a stimulatory component in the sebum has an excitatory function (an action for increasing the oleic acid concentration) of the cell such as epidermal keratinocyte. This suggests that it is a useful means for inhibiting parakeratosis and shrinkage of the pore to inhibit the cells from being excited by the sebum using the antagonist to the excitatory cell receptor. [Preparation of sample]

An aqueous solution containing 3% each of GABA, glycine, D-glutamic acid and L-glutamic acid was prepared, and pH of each solution was adjusted to nutrality, if necessary. An aqueous solution containing 10 mM each of GABA, bicuculline methobromide (antagonist to GABA receptor type A), MK-801, muscimol, isogubacin, ATP, suramin, PPADS and TNP-ATP was also prepared.

### [Test on parakeratosis inhibitory action]

A 3% or 30% oleic acid solution (100 µL, solvent: ethanol) was applied on the back of a hairless mouse. Thereafter, 100 µL of sample solution or reference solution was applied. This procedure was repeated for 3 days, and the keratin layer on the back was peeled with a tape. The nucleus of the keratin layer was stained with hematoxylin, the number of nuclear cells was counted under a microscope, and the results were evaluated in four grades of 1 to 4. The results of application of the 3% oleic acid solution, and the results of application of the 30% oleic acid solution are shown in Tables 2 and 3, respectively. Since the evaluation criteria are different between application of the 3% oleic acid solution and application of the 30% oleic acid solution, respective results are expressed by relative evaluations.

**Table 2 Application Test of 30% Oleic Acid Solution**

| Sample (3% Solution) | Incidence of Parakeratosis (Average of 4 mice) |
|---|---|
| Water (Reference) | 2.5 |
| GABA | 1.5 |
| Glycine | 1.3 |
| D-Glutamic Acid | 2.0 |
| L-Glutamic Acid | 3.0 |

**Table 3 Application Test of 3% Oleic Acid Solution**

| Sample (10mM) | Incidence of Parakeratosis (Average of 4 mice) |
|---|---|
| Water (Reference) | 2.0 |
| GABA | 1.5 |
| GABA + bicuculline methobromide | 2.5 |
| MK-801 | 1.0 |
| Muscimol | 1.3 |
| Isogubacin | 1.3 |
| ATP | 2.3 |
| Suramin | 1.5 |
| PPADS | 1.8 |
| TNP-ATP | 1.5 |

The results in Tables 2 and 3 show that L-glutamic acid as an agonist to the glutamic acid receptor (NMDA receptor) that is an excitatory cell receptor worsens parakeratosis caused by oleic acid, while MK-801 and D-glutamic acid as antagonists of the glutamic acid receptor (NMDA receptor) improved parakeratosis caused by oleic acid.

ATP as an agonist to the ATP receptor (P2X receptor) that is an excitatory cell receptor worsens parakeratosis caused by oleic acid, while suramin, PPADS and TNP-ATP that are antagonists of the ATP receptor (P2X receptor) improved parakeratosis caused by oleic acid.

GABA, muscimol and isogubacin as the agonists to the GABA receptor (GABA receptor type A) as inhibitory cell receptors improved parakeratosis caused by oleic acid, while bicuculline methobromide as the antagonist to the GABA receptor (GABA receptor type A) inhibited parakeratosis inhibitory action by oleic acid.

Glycine as the agonist to the glycine receptor as an inhibitory cell receptor improved parakeratosis caused by oleic acid.

It was shown as described above that the antagonist to the excitatory cell receptor have parakeratosis inhibitory actions by oleic acid.

### [Test on pore-shrinking action]

The cheek of healthy males were subjected to the test for applying the sample solution twice a day for 1 month. The sample solutions (glycine, GABA and D-glutamic acid) each had a concentration of 3%. Replicas were sampled before and after the completion of the test, and the changes of the shape of the pores at the same site were observed under a three-dimensional laser scan microscope. The size of the pore was visually evaluated in 13 grades of 1 to 13. The difference of the scores before and after the test was calculated and used for evaluating each agent. The results are shown in Table 4.

**Table 4**

| Sample | Decision of Replica of Pores (Average of n = 5) |
|---|---|
| Glycine | -1.2 |
| GABA | -1.0 |
| D-Glutamic Acid | -0.4 |

It was confirmed from the results in Table 4 that glycine, GABA and D-glutamic acid as the antagonists to the excitatory cell receptors have excellent pore-shrinking effects.

Examples of the composition for external uses of the invention are shown below. Any types of the composition in Examples showed excellent effects as the parakeratosis inhibitory skin preparation for external use and pore-shrinking skin preparation for external use.

### [Comparative Example 1: Cream]

| (Prescription) | Blend Ratio (% by mass) |
|---|---|
| Stearic Acid | 5.0 |
| Stearyl Alcohol | 4.0 |
| Isopropyl Myristate | 18.0 |
| Glycerin Monostearate Ester | 3.0 |
| Propyleneglycol | 10.0 |
| Glycine | 0.5 |
| Potassium Hydroxide | 0.3 |
| Sodium Hydrogen Sulfite | 0.01 |
| Antiseptics | appropriate amount |
| Perfume | appropriate amount |
| Ion-Exchanged Water | balance |

### (manufacturing method)

Propyleneglycol, glycine and potassium hydroxide were dissolved in ion-exchanged water, and kept at 70°C by heating (aqueous phase). The other components were mixed and melted at 70°C by heating (oil phase). The oil phase was slowly added to the aqueous phase, and was allowed to disperse by keeping the temperature for a while after adding all the oily phase. The mixture was uniformly emulsified with a homomixer, and was cooled to 30°C with thorough stirring.

### [Comparative Example 2: Cream]

| (Prescription) | Blend Ratio (% by mass) |
|---|---|
| Solid Paraffin | 5.0 |
| Beeswax | 10.0 |
| Vaseline | 15.0 |
| Liquid Paraffin | 41.0 |
| Glycerin Monostearate Ester | 2.0 |
| Polyoxyethylene (20 mol) Sorbitan Monolaurate Ester | 2.0 |
| Soap Powder | 0.1 |
| Borax | 0.2 |
| PPADS·4Na | 0.05 |
| Sodium Hydrogen Sulfite | 0.03 |
| Ethylparaben | 0.3 |
| Perfume | appropriate amount |
| Ion-Exchanged Water | balance |

### (Preparation method)

Soap powder, borax and PPADS·4Na were added in ion-exchanged water, and were dissolved by heating at 70°C (aqueous phase). The other components were mixed and melted at 70°C by heating (oil phase). The oil phase was slowly added to the aqueous phase to allow the two phases to react. After the completion of the reaction, the mixture was uniformly emulsified with a homomixer, and was cooled to 30°C with thorough stirring after the emulsification.

### [Comparative Example 3: Lotion]

| (Prescription) | Blend Ratio (% by mass) |
|---|---|
| Stearic Acid | 2.5 |
| Cetyl Alcohol | 1.5 |
| Vaseline | 5.0 |
| Liquid Paraffin | 10.0 |
| Polyoxyethylene (10 mol) Monooleateate Ester | 2.0 |
| Polyethylene Glycol 1500 | 3.0 |
| Triethanolamine | 1.0 |
| Carboxyvinyl Polymer | 0.05 |

| (Trade name: Carbopole 941, manufacfactured by B. F. Goodrich Chemical Co.) | |
|---|---|
| GABA | 0.5 |
| Potassium Hydroxide | 0.4 |
| Sodium Hydrogen sulfite | 0.01 |
| Ethylparaben | 0.3 |
| Perfume | appropriate amount |
| Ion-Exchanged Water | balance |

### (Preparation method)

Carboxyvinyl polymer, GABA and potassium hydroxide were dissolved in a small volume of ion-exchanged water (phase A). Polyethyleneglycol 1500 and triethanolamine were added to the remaining ion-exchanged water, and dissolved by heating at 70°C (aqueous phase). The other components were mixed and melted at 70°C by heating (oil phase). The oil phase was added to the aqueous phase for pre-emulsification, and phase A was added to the emulsion to uniformly emulsify with a homomixer, followed by cooling to 30°C with thorough stirring after the emulsification.

### [Comparative Example 4: Jelly]

| (Prescription) | Blend Ratio (% by mass) |
|---|---|
| 95% Ethyl Alcohol | 10.0 |
| Dipropyleneglycol | 15.0 |
| Polyoxyethylene (50 mol) Oleyl Alcohol Ether | 2.0 |
| Carboxyvinyl Polymer | 1.0 |

| (trade name: Carbopole 940, manuffactured by B.F. Goodrich Chemical Co.) | |
|---|---|
| Sodium Hydroxide | 1.0 |
| L-Arginine | 0.1 |
| Glycine | 5.0 |
| Dimorpholinopyridazinon | 0.05 |
| EDTA·3Na·2H₂O | 0.05 |
| Methylparaben | 0.2 |
| Perfume | appropriate amount |
| Ion-Exchanged Water | balance |

### (Preparation method)

Carbopole 940 was uniformly dissolved in ion-exchanged water, while polyoxyethylene (50 mol) oleyl alcohol ether was dissolved in 95% ethanol and was added to the aqueous phase. After adding the other components, the solution was neutralized with sodium hydroxide and L-arginine to viscosity the solution

### [Comparative Example 5: Beauty liquid]

| (Prescription) | Blend Ratio (% by mass) |
|---|---|
| (Phase A) | |
| Ethyl alcohol (95%) | 10.0 |
| Polyoxyethylene (20 mol) octyldodecanol | 1.0 |
| Pantotenyl Ethylether | 0.1 |
| Methylparaben | 0.15 |

| (Phase B) | |
|---|---|
| Potassium Hydroxide | 0.1 |

| (Phase C) | |
|---|---|
| Glycerin | 5.0 |
| Dipropyleneglycol | 10.0 |
| MK-801 | 0.03 |
| Carnoxyvinyl Polymer | 0.2 |

| (trade name: Carbopole 940, manufactured by B.F. Goodrich Chemical Co.) | |
|---|---|
| Purified Water | balance |

### (Preparation method)

Phase A and Phase C each was uniformly dissolved, and phase A was added to phase C for solubilization. Then, phase B was added to the mixture, which was filled in a vessel.

### [Comparative Example 6: Lotion]

| (Prescription) | Blend Ratio (% by mass) |
|---|---|
| Glycerin | 5.0 |
| 1,3-Butyleneglycol | 3.0 |
| Dipropyleneglycol | 2.0 |
| Sodium Succinate | 0.1 |
| Succinic Acid | 0.07 |
| Suramin | 0.01 |
| Ethanol | 5.0 |
| Methylparaben | 0.15 |
| Perfume | appropriate amount |
| EDTA·3Na·2H₂O | 0.1 |
| Purified Water | balance |

### (Preparation method)

Methylparaben and perfume were added to and dissolved in ethanol (ethanol phase). The alcohol phase and the other components were added and solubilized in purified water, followed by filling in a vessel.

### [Example 7: Lotion]

| (Prescription) | Blend Ratio (% by mass) |
|---|---|
| Glycerin | 2.0 |
| Dipropyleneglycol | 5.0 |
| Sodium Citrate | 0.08 |
| Citric Acid | 0.02 |
| D-glutamic Acid | 0.5 |
| KOH | 0.01 |
| Extract of Pyrola japonica (ichiyaakusou) (extracted with Ethanol) | 0.05 |
| Extract of Akebia quinata (extractedd with 1, 3-butyleneglycol) | |
| | 0.05 |
| Ethanol | 2.0 |
| Phenoxy Ethanol | 0.05 |
| Polyoxyethylene Polypropylene Decyyltetradecylether | 0.02 |
| Perfume | appropriate amount |
| EDTA·3Na·2H₂O | 0.1 |
| Purified Water | balance |

### (preparation method)

The other components were added and solubilized in purified water, and the solution was filled in a vessel.

### [Comparative Example 8: Lotion (micro-emulsion)]

| (Prescription) | Blend Ratio (% by mass) |
|---|---|
| 1,3-butyleneglycol | 6.0 |
| Glycerin | 5.0 |
| Polyethyleneglycol 4000 | 3.0 |
| Olive Oil | 0.5 |
| Polyoxyethylene (20 mol) Sorbitan Monostearate | 1.5 |
| Polyoxyethylene (5 mol) Oleyl Monoalcohol Ether | 0.3 |
| Ethanol | 1.0 |
| Muscimol | 0.05 |
| Citric Acid | 0.07 |
| Sodium Citrate | 0.03 |
| Methylparaben | 0.15 |
| Perfume | appropriate amount |
| Purified Water | balance |

### (Preparation method)

1,3-butyleneglycol, glycerin, polyethyleneglycol 4000, muscimol, citric acid and sodium citrate were dissolved in purified water (aqueous phase). Olive oil, polyoxyethylene (20 mol) sorbitan monostearate, polyoxyethylene (5 mol) oleyl alcohol ether, methylparaben and perfume were dissolved in ethanol (alcohol phase). The alcohol phase was added to the aqueous phase to prepare a micro-emulsion, which was filled in a vessel

### [Comparative Example 9: Powder lotion]

| (Prescription) | Blend Ratio (% by mass) |
|---|---|
| Ethanol | 15.0 |
| Glycerin | 2.0 |
| 1,3-butyleneglycol | 2.0 |
| TNP-ATP | 0.01 |
| L-arginine | 0.1 |
| Iron Oxide | 0.15 |
| Zinc Oxide | 0.5 |
| Kaolin | 2.0 |
| HEDTA·3Na | 0.2 |
| Menthol | 0.2 |
| Perfume | appropriate amount |
| Purified Water | balance |

### (Preparation method)

Glycerin, 1,3-butyleneglycol, TNP-ATP and L-arginine were dissolved in water (aqueous phase). Menthol and perfume were dissolved in ethanol (alcohol phase). The alcohol phase was added to the aqueous phase, and iron oxide, zinc oxide and kaolin were added to the mixed solution, which was homogenized with a mixer and filled in a vessel.

### [Comparative Example 10: Emulsified foundation]

| (Prescription) | Blend Ratio (% by mass) |
|---|---|
| (Powder Part) | |
| Titanium Dioxide | 10.3 |
| Sericite | 5.4 |
| Kaolin | 3.0 |
| Yellow Iron Oxide | 0.8 |
| Iron Red | 0.3 |
| Black Iron Oxide | 0.2 |

| (Oil Phase) | |
|---|---|
| Decamethylcyclopentane Siloxane | 11.5 |
| Liquid Paraffin | 4.5 |
| Polyoxyethylene-modified dimethylpoolysiloxane | 4.0 |

| (Aqueous Phase) | |
|---|---|
| Purified Water | balance |
| 1,3-butyleneglycol | 4.5 |
| Isogubacin | 0.01 |
| Sorbitan Sesquioleate Ester | 3.0 |
| Antiseptics | appropriate amount |
| Perfume | appropriate amount |

### (Preparation method)

After the aqueous phase was heated and stirred, the sufficiently mixed and pulverized powder part was added to the aqueous phase, and the mixture was homogenized with a homomixer. The perfume was added while stirring, and the mixture was cooled to room temperature.

### Industrial Applicability

The antagonist to the excitatory cell receptor of the invention inhibits parakeratosis caused by stimulatory components in the sebum at a site particularly susceptible to the sebum such as around the pores. Accordingly, the antagonist functions as a parakeratosis inhibitor and pore-shrinking agent that expresses excellent effects such as prevention of conical expansion of the pore around the pores, inhibition of conspicuous conical pore structure and shrinkage of the pore that enable a healthy skin state with inconspicuous pores to be maintained. An excellent parakeratosis inhibitor as an external use skin preparation and an excellent pore-shrinking agent as an external use skin preparation can be obtained by permitting the agents to contain such compounds as effective ingredients.

## Claims

1. A preparation for skin for therapeutic use in inhibiting parakeratosis by external use, comprising an antagonist to an excitatory cell receptor,
wherein the antagonist is D-glutamic acid, and wherein the excitatory cell receptor is an N-methyl-D-aspartic acid receptor.

2. A non-therapeutic use of a preparation for skin for external use for inhibiting parakeratosis or for shrinking pores, comprising an antagonist to an excitatory cell receptor, wherein the antagonist is D-glutamic acid, and wherein the excitatory cell receptor is an N-methyl-D-aspartic acid receptor.

## Patentansprüche

1. Hautpräparat für die therapeutische Anwendung zur Inhibierung von Parakeratose durch äußerliche Anwendung, umfassend einen Antagonisten zu einem exzitatorischen Zellrezeptor,
wobei der Antagonist D-Glutaminsäure ist, und wobei der exzitatorische Zellrezeptor ein N-Methyl-D-asparaginsäure-Rezeptor ist.

2. Nichttherapeutische Verwendung eines Hautpräparats für die äußerliche Anwendung zur Inhibierung von Parakeratose oder zur Schrumpfung von Poren, umfassend einen Antagonisten zu einem exzitatorischen Zellrezeptor, wobei der Antagonist D-Glutaminsäure ist, und wobei der exzitatorische Zellrezeptor ein N-Methyl-D-asparaginsäure-Rezeptor ist.

## Revendications

1. Préparation pour la peau pour une utilisation thérapeutique d'inhibition de la parakératose à usage externe, comprenant un antagoniste à un récepteur cellulaire excitateur
où l'antagoniste est un acide D-glutamique, et où le récepteur cellulaire excitateur est un récepteur d'acide N-méthyl-D-aspartique.

2. Utilisation no-thérapeutique d'une préparation pour la peau à usage externe pour inhiber la parakératose ou pour rétrécir les pores, comprenant un antagoniste à un récepteur cellulaire excitateur, où l'antagoniste est un acide D-glutamique, et où le récepteur cellulaire excitateur est un récepteur d'acide N-méthyl-D-aspartique.
